(19) Europäisches Patentamt
European Patent Office
Office européen des brevets

(11) EP 2 540 294 B1

(12) EUROPEAN PATENT SPECIFICATION

(45) Date of publication and mention of the grant of the patent:
**03.08.2016 Bulletin 2016/31**

(21) Application number: **11744792.0**

(22) Date of filing: **21.02.2011**

(51) Int Cl.:
***A61K 31/403*** (2006.01) ***A61K 9/16*** (2006.01)
***A61K 9/22*** (2006.01) ***A61K 31/4745*** (2006.01)
***A61K 47/10*** (2006.01) ***A61K 47/26*** (2006.01)
***A61K 47/32*** (2006.01) ***A61K 47/36*** (2006.01)
***A61K 47/38*** (2006.01)

(86) International application number:
**PCT/JP2011/053644**

(87) International publication number:
**WO 2011/102506 (25.08.2011 Gazette 2011/34)**

# (54) SUSTAINED-RELEASE SOLID PREPARATION FOR ORAL USE

FESTSTOFFPRÄPARAT MIT VERZÖGERTER FREISETZUNG ZUR ORALEN VERABREICHUNG

PRÉPARATION SOLIDE À LIBÉRATION PROLONGÉE POUR USAGE ORAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.02.2010 JP 2010035884**

(43) Date of publication of application:
**02.01.2013 Bulletin 2013/01**

(73) Proprietor: **Daiichi Sankyo Company, Limited**
**Tokyo 103-8426 (JP)**

(72) Inventors:
- **KANAMARU, Taro**
**Tokyo 140-8710 (JP)**
- **TAJIRI, Shinichiro**
**Tokyo 140-8710 (JP)**

(74) Representative: **Fairbairn, Angus Chisholm**
**Marks & Clerk LLP**
**90 Long Acre**
**London WC2E 9RA (GB)**

(56) References cited:
**EP-A1- 2 140 867**
**WO-A1-03/000657**
**WO-A1-03/000680**
**WO-A1-03/016302**
**WO-A1-2004/058715**
**WO-A1-2008/041553**
**WO-A1-2008/100107**
**WO-A2-2007/031887**
**WO-A2-2009/047802**
**WO-A2-2009/057138**
**JP-A- 2001 270 821**
**JP-A- 2001 513 752**
**JP-A- 2001 522 794**
**JP-A- 2003 507 413**
**JP-A- 2008 169 173**
**JP-A- 2009 500 317**
**JP-A- 2009 532 462**
**JP-A- 2009 535 351**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a sustained-release solid preparation that reliably exhibits its main pharmacological effect when orally administered once or twice a day.

### Background Art

Sustained-release preparations for the adjustment of blood concentrations of drugs are highly useful in terms of separation between the main pharmacological effect and adverse reaction, improvement in compliance (e.g., the number of doses reduced by improvement in prolonged efficacy), medical economy, etc. In this regard, some techniques have been reported for sustained-release preparations. Meanwhile, since compounds exhibiting the main pharmacological effect have diverse chemical properties, some sustained release techniques, albeit still insufficient, adaptable to the diverse chemical properties of these compounds have been reported (see e.g., Patent Documents 1 and 2).

The properties of a drug itself can be classified broadly into neutral, acidic, and basic properties. Among others, solubility (degree of solubility) in water differs greatly between compounds. Low water-soluble compounds have many disadvantages in the design of preparations to improve dissolution properties. Acidic drugs refer to acidic compounds that are acidic in the free form (whose acidic group does not constitute a salt such as an alkali- or amine-addition salt). Acidic drugs are disadvantageously low soluble in an acidic solutions, for example, in the upper gastrointestinal tract such as the stomach. A salt (alkali- or amine-addition salt) of an acidic compound disadvantageously becomes a low soluble free acid in an acidic solution. Alternatively, basic drugs refer to basic compounds that are basic in the free form (whose basic group does not constitute a salt such as acid-addition salt) and are known to exhibit favorable solubility in strongly acidic aqueous solutions, but exhibit reduced solubility in neutral aqueous solutions such as a neutral buffer. Specifically, basic drugs, when orally administered, exhibit favorable solubility in the stomach, which is acidic. Their solubility, however, is greatly reduced in the lower gastrointestinal tract such as the large intestine, which is neutral with little water, probably leading to a reduced absorption rate of the drug.

For example, a challenge for the design of sustained-release preparations for oral administration containing a basic drug is dose dumping of the drug when the preparation collapses due to mechanical stress resulting from the presence of food in the acidic environment of the upper gastrointestinal tract exhibiting high water-solubility, gastrointestinal motility, and so on. Furthermore, preparation strength may be enhanced by, for example, an increased amount of a sustained-release agent in order to avoid dose dumping of the drug. In such a case, still, the challenge for a sustained-release preparation containing a basic drug whose water solubility is reduced in the neutral region is to improve the dissolution properties of the preparation in the lower gastrointestinal tract and maintain drug absorption. No previous technique for sustained-release preparations containing a basic drug can simultaneously achieve, at satisfactory levels, avoidance of dose dumping of the drug in an acidic environment such as the upper gastrointestinal tract and prolonged dissolution in the lower gastrointestinal tract, which is a neutral environment.

### Citation List

### Patent Document

Patent Document 1: National Publication of International Patent Application No. 2006/507216
Patent Document 2: National Publication of International Patent Application No. 2004/518676

WO2007031887 A2 discloses controlled release tablets of metformin hydrochloride comprising 1.9% by weight povidone, Carbopol 714, and carmellose sodium.

### Summary of Invention

### Technical Problem

An object of the present invention is to avoid dose dumping of a drug caused by mechanical stress resulting from gastrointestinal motility in the presence of food in the acidic environment of the upper gastrointestinal tract, particularly, the stomach, and improve the dissolution properties of the drug in the lower gastrointestinal tract, which is the neutral region, and thereby provide a sustained-release preparation for oral administration containing, as a principal

pharmaceutically active ingredient, a drug that reliably exhibits its main pharmacological effect when orally administered once or twice a day.

Solution to Problem

As a result of conducting studies on the formulation of sustained-release preparations for oral administration, the present inventors have found that a sustained-release solid preparation containing a pharmacologically active drug, a carboxyvinyl polymer, povidone, and a swelling agent can avoid dose dumping of the drug under an acidic environment and can be improved in its dissolution properties in the neutral region. Based on this finding, the present invention has been completed.

Specifically, the present invention provides the following (1) to (11) :

(1) A sustained-release solid preparation containing (A) a pharmacologically active drug, (B) a carboxyvinyl polymer, (C) povidone, and (D) carmellose sodium or xanthan gum, and (E)a sugar alcohol, wherein the content of the component (C) in the preparation is 10 to 70% by weight.
(2) The preparation according to (1), wherein the component (D) in the preparation is carmellose sodium.
(3) The preparation according to (1) or (2), wherein the content of the component (A) in the preparation is 5 to 35% by weight.
(4) The preparation according to any one of (1) to (3), wherein the content of the component (B) in the preparation is 5 to 30% by weight.
(5) The preparation according to any one of (1) to (4), wherein the content of the component (D) in the preparation is 5 to 15% by weight.
(6) The preparation according to (1) to 5), wherein the content of the sugar alcohol in the preparation is 10 to 40% by weight.
(7) The preparation according to (1) to (6), wherein the sugar alcohol is mannitol, xylitol, or erythritol.
(8) The preparation according to (1) or (6), wherein the sugar alcohol is xylitol.
(9) The preparation according to any one of (1) to (8), wherein the component (A) is a basic drug.
(10) The preparation according to any one of (1) to (8), wherein the component (A) is a compound selected from the group consisting of

(±)-1-(carbazol-4-yloxy)-3-[[2-(o-methoxyphenoxy)ethyl]amino]-2-propanol,
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide, and
$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide

or a pharmacologically acceptable salt thereof, or a hydrate thereof.
(11) The preparation according to any one of (1) to (10), wherein the dosage form is a tablet.

Advantageous Effects of the Invention

The present invention can provide a sustained-release pharmaceutical composition for oral administration containing a pharmacologically active drug. Thus, the present invention provides, for example, a sustained-release solid preparation for oral use having a prolonged effect, which contains activated blood coagulation factor X (FXa) inhibitor compound (1) as a pharmaceutically active ingredient. The sustained-release pharmaceutical composition of the present invention has a favorable hydration rate and swelling rate in an acidic solution and has a favorable tablet strength that prevents dose dumping. In addition, the preparation of the present invention has favorable dissolution properties in a neutral solution. Thus, the sustained-release pharmaceutical composition of the present invention is effective for maintaining a prolonged dissolution of the pharmacologically active drug contained therein from the duodenum through the small intestine to the lower gastrointestinal tract.

Brief Description of the Drawings

[Figure 1] Figure 1 is a diagram showing time-dependent change in the dissolution rate of a drug up to 2 hours later at paddle rotation rates of 200 rpm and 50 rpm when tablets of Comparative Example 1 described in Table 1 were tested by a dissolution test in an acidic solution (the paddle method, 0.01 N hydrochloric acid, 900 mL) at paddle

rotation rates of 200 rpm and 50 rpm.

[Figure 2] Figure 2 is a diagram showing time-dependent change in the dissolution rate of a drug up to 2 hours later at paddle rotation rates of 200 rpm and 50 rpm when tablets of Comparative Example 2 described in Table 1 were tested by a dissolution test in an acidic solution (the paddle method, 0.01 N hydrochloric acid, 900 mL) at paddle rotation rates of 200 rpm and 50 rpm.

[Figure 3] Figure 3 is a diagram showing time-dependent change in the dissolution rate of a drug up to 2 hours later at paddle rotation rates of 200 rpm and 50 rpm when tablets of Comparative Example 3 described in Table 1 were tested by a dissolution test in an acidic solution (the paddle method, 0.01 N hydrochloric acid, 900 mL) at paddle rotation rates of 200 rpm and 50 rpm.

[Figure 4] Figure 4 is a diagram showing time-dependent change in the dissolution rate of a drug up to 2 hours later at paddle rotation rates of 200 rpm and 50 rpm when tablets having formulation 1 described in Table 1 were tested by a dissolution test in an acidic solution (the paddle method, 0.01 N hydrochloric acid, 900 mL) at paddle rotation rates of 200 rpm and 50 rpm.

[Figure 5] Figure 5 is a diagram showing time-dependent change in the dissolution rate of a drug when tablets having formulation 1 described in Table 1 were tested by a dissolution test in a neutral solution (the paddle method, phosphate buffer, pH 6.8, 900 mL) at a paddle rotation rate of 50 rpm.

[Figure 6] Figure 6 is a diagram showing time-dependent change in the dissolution rate of a drug up to 2 hours later at paddle rotation rates of 200 rpm and 50 rpm when tablets having formulation 2a described in Table 4 were tested by a dissolution test in an acidic solution (the paddle method, 0.01 N hydrochloric acid, 900 mL) at paddle rotation rates of 200 rpm and 50 rpm.

[Figure 7] Figure 7 is a diagram showing time-dependent change in the dissolution rate of a drug up to 2 hours later at paddle rotation rates of 200 rpm and 50 rpm when tablets having formulation 2b described in Table 4 were tested by a dissolution test in an acidic solution (the paddle method, 0.01 N hydrochloric acid, 900 mL) at paddle rotation rates of 200 rpm and 50 rpm.

[Figure 8] Figure 8 is a diagram showing time-dependent change in the dissolution rate of a drug up to 2 hours later at paddle rotation rates of 200 rpm and 50 rpm when tablets having formulation 3 described in Table 6 were tested by a dissolution test in an acidic solution (the paddle method, 0.01 N hydrochloric acid, 900 mL) at paddle rotation rates of 200 rpm and 50 rpm.

[Figure 9] Figure 9 is a diagram showing time-dependent change in the dissolution rate of a drug when a swelling base-unsupplemented formulation (formulation 4) described in Table 8 was tested by a dissolution test in a neutral solution (the paddle method, phosphate buffer, pH 6.8, 900 mL) at a paddle rotation rate of 50 rpm.

[Figure 10] Figure 10 is a diagram showing time-dependent change in the dissolution rate of a drug when formulation 5 described in Table 8 was tested by a dissolution test in a neutral solution (the paddle method, phosphate buffer, pH 6.8, 900 mL) at a paddle rotation rate of 50 rpm.

[Figure 11] Figure 11 is a diagram showing time-dependent change in the dissolution rate of a drug when formulation 6 described in Table 8 was tested by a dissolution test in a neutral solution (the paddle method, phosphate buffer, pH 6.8, 900 mL) at a paddle rotation rate of 50 rpm.

[Figure 12] Figure 12 is a diagram showing time-dependent change in the dissolution rate of a drug when formulation 7 described in Table 8 was tested by a dissolution test in a neutral solution (the paddle method, phosphate buffer, pH 6.8, 900 mL) at a paddle rotation rate of 50 rpm.

[Figure 13] Figure 13 is a diagram showing change in the plasma concentration of a drug when tablets having formulations 4' and 5' described in Table 8 (dose: 20 mg/head) and an aqueous bulk solution (dose: 10 mg/head) were separately administered to the large intestine of each fasted dog.

## Description of Embodiments

Hereinafter, the present invention will be described in detail.

In the present specification, "acidic solution" means an acidic dissolution test medium used for evaluation of dissolution properties in the upper gastrointestinal tract such as the stomach. Non-limiting examples of the acidic dissolution test medium can include: the JP 1st dissolution test fluid described in the Japanese Pharmacopoeia; and USP 0.1 N hydrochloric acid, 0.01 N hydrochloric acid, and Simulated Gastric Fluid without Enzyme described in the United States Pharmacopoeia.

In the present specification, "neutral solution" means a neutral dissolution test medium used for evaluation of drug dissolution properties in the small intestine, the large intestine, or the like. Non-limiting examples of the neutral dissolution test medium can include dissolution test media (pH 6.8) such as: the JP 2nd dissolution test fluid and phosphate buffer (pH 6.8) described in the Japanese Pharmacopoeia; USP Phosphate Buffer (pH 6.8) and Simulated Intestinal Fluid without Enzyme described in the United States Pharmacopoeia; and Phosphate Buffer Solution (pH 6.8) described in the European Pharmacopoeia.

The aforementioned dissolution test medium is prepared through methods described in the corresponding pharmacopoeia or the like of each country. When the employed dissolution test medium is a buffer solution, variation of the pH of the test medium is preferably within ±0.05 of the pH defined for each dissolution medium.

Examples of the paddle method using an acidic dissolution medium for the evaluation of dissolution properties of the sustained-release solid preparation of the present invention in the upper gastrointestinal tract can include a method in which a dissolution test is conducted by the paddle method at rotation rates of 50 rpm and 200 rpm at 37 ± 0.5°C for 2 hours in 0.01 N hydrochloric acid (900 mL). As described above, when the pharmacologically active drug in the preparation is a basic drug, dose dumping of the drug becomes a problem because the preparation collapses due to mechanical stress resulting from the presence of food in the acidic environment of the upper gastrointestinal tract exhibiting high water-solubility, gastrointestinal motility, and so on. Thus, the average percentage dissolution of the pharmacologically active drug in the acidic dissolution test medium is preferably a value that allows preparation strength to be maintained and the dissolution rate to be kept within a predetermined range at the rotation rates of 200 rpm and/or 50 rpm in the paddle method. The average percentage dissolution of the pharmacologically active drug in the dissolution test medium after 2 hours is preferably 50% or lower, more preferably 40% or lower, even more preferably 30% or lower, at the rotation rates of 200 rpm and/or 50 rpm in the paddle method. Moreover, when the preparation is subjected to the dissolution test method for 2 hours, the difference in average percentage dissolution (value at the rotation rate of 200 rpm in the paddle method - value at the rotation rate of 50 rpm in the paddle method) of the pharmacologically active drug in the dissolution test medium is preferably 15% or lower, more preferably 10% or lower, even more preferably 5% or lower. Moreover, the average percentage dissolution ratio (value at the rotation rate of 200 rpm in the paddle method / value at the rotation rate of 50 rpm in the paddle method) of the pharmacologically active drug in the dissolution test medium after 2 hours is preferably 2.0 or lower, more preferably 1.5 or lower, particularly preferably 1.3 or lower.

Examples of the paddle method using a neutral dissolution medium for the evaluation of dissolution properties of the sustained-release solid preparation of the present invention in the neutral region can include a method in which a dissolution test is conducted by the paddle method at a rotation rate of 50 rpm at 37 ± 0.5°C in phosphate buffer (pH 6.8; 900 mL). In the dissolution test medium, the pharmacologically active drug preferably exhibits a prolonged dissolution rate as an average percentage dissolution even 12 hours after the start of the dissolution test. More preferably, the pharmacologically active drug exhibits a prolonged dissolution rate even 12 hours after the start of the dissolution test and exhibits an average percentage dissolution of 20% or more, even more preferably 30% or more.

USP Apparatus 3 (Bio-Dis method), which is a dissolution test method under conditions close to the environment of the human gastrointestinal tract, may be used for the dissolution test.

The concentration of the drug in a solution can be determined using the UV method or the like. The average percentage dissolution and dissolution time of the pharmacologically active drug in the dissolution test medium can be calculated.

As used herein, "average percentage dissolution" refers to the average of percentage dissolution values obtained from at least 2, preferably 6, more preferably 12 solid preparation samples for each type of solid preparation.

Moreover, the dissolution properties of the pharmacologically active drug from the sustained-release solid preparation of the present invention can be confirmed using an *in vivo* animal test. Examples of the *in vivo* animal test can include *in vivo* absorption property evaluation using dogs. In general, an orally administered preparation allegedly passes through the stomach and the small intestine and then stays for a long time in the large intestine. Therefore, for sustained-release preparations having a long dissolution time, it is very important to prolong drug release in the large intestine in which the preparation stays for a long time. Examples of a method for confirming the absorption properties of the pharmacologically active drug contained in the preparation in the large intestine can include canine large intestinal absorption property evaluation in which the preparation is directly administered into the canine large intestine. Specifically, the absorption properties in the canine large intestine can be confirmed from blood concentrations measured after administration, and evaluated based on the relative bioavailability (BA) or the like of each tablet from their ratios to those of an orally administered aqueous solution of the pharmacologically active drug.

In the present specification, the "pharmacologically active drug" is preferably a relatively low water-soluble drug that exhibits the main pharmacological effect of the formulation of the preparation. A neutral compound of the pharmacologically active drug means a compound that does not have a group dissociable by ionization in the acidic or basic state in its molecule. Moreover, an acidic compound means a drug having an acidic group typified by a carboxy group, a phenolic hydroxy group, a phosphoric acid group, a sulfonic acid group, a tetrazolyl group, or the like. Furthermore, a basic drug means a drug having a basic nitrogen atom typified by an amino group, a piperidinyl group, a piperazinyl group, or the like in its molecule. In the present invention, particularly, a basic drug is preferred. The basic drug has physicochemical properties in which the degree of solubility is lower in the neutral state (7.5 > pH > 5) in the small intestine or the large intestine than that in the acidic state (pH ≤ 2).

As described above, the basic drug according to the present specification refers to a drug having a degree of solubility that is lower in the neutral state than in the acidic state. Non-limiting examples of the rate of this reduction in the degree of solubility in the neutral state can include the following ranges:

preferably, (degree of solubility in the neutral state) / (degree of solubility in the acidic state) in the range of 0.00001 to 0.6;

more preferably, (degree of solubility in the neutral state) / (degree of solubility in the acidic state) in the range of 0.001 to 0.5; and

even more preferably, (degree of solubility in the neutral state) / (degree of solubility in the acidic state) in the range of 0.01 to 0.1.

In the present specification, the "basic drug" preferably has a degree of solubility in the range of 1 to 500 mg/ml in the acidic region (the JP 1st dissolution test fluid; pH 1.2, 20 $\pm$ 5°C) and a degree of solubility in the range of 0.01 to 3000 $\mu$g/ml in the neutral region (the JP 2nd dissolution test fluid; pH 6.8, 20 $\pm$ 5°C).

More preferred is a basic drug having a degree of solubility in the range of 1 to 500 mg/ml in the acidic region (the JP 1st dissolution test fluid; pH 1.2, 20 $\pm$ 5°C) and a degree of solubility in the range of 10 to 500 $\mu$g/ml in the neutral region (the JP 2nd dissolution test fluid; pH 6.8, 20 $\pm$ 5°C). Moreover, the absolute value of the degree of solubility in the drug is preferably the lowest degree of solubility reduced to 3 mg/ml or lower, more preferably 1 mg/ml or lower, even more preferably 0.5 mg/ml or lower, in the neutral state (in the range of 7.5 > pH > 5).

Specific examples of the "pharmacologically active drug" can include anticoagulant agents.

The anticoagulant agent is preferably an activated blood coagulation factor X (FXa) inhibitor having a basic or weakly basic substituent. Specific examples of the FXa inhibitor can include the following (a) to (1) :

(a) Darexaban Maleate (tanexaban) (N-[2-hydroxy-6-(4-methoxybenzamido)phenyl]-4-(4-methyl-1,4-diazepan-1-yl)benzamide) [See PFSB/ELD No. 1111-1 (November 11, 2010); Pre-publication copy, Proposed INN: List 101; Research and development pipeline. Yamanouchi Pharmaceutical Co Ltd. Company World Wide Web site, 11 Feb 2004];

(b) rivaroxaban (5-chloro-N-({(5S)-2-oxo-3-[4-(3-oxo-4-morpholinyl)phenyl]-1,3-oxazolidin-5-yl}methyl)-2-thiophenecarboxamide) [See WFO Drug Information, Vol. 18, No. 3, 2004, page 260; Susanne R, et al, J. Med. Chem., 2005, 48, 5900-5908; D. Kubitza et al, Multiple dose escalation study investigating the pharmacodynamics, safety, and pharmacokinetic of Bay59-7939, an oral, direct Factor Xa inhibitor, in healthy male subjects. Blood, 2003, 102; Abstract 3004];

(c) apixaban (1-(4-methoxyphenyl)-7-oxo-6-[4-(2-oxopiperidin-1-yl)phenyl]-4,5,6,7-tetrahydro-1H-pyrazolo[3,4-c]pyridine-3-carboxamide) [See WFO Drug Information, Vol. 20, No. 1, 2006, page 38; Pinto DJP, Orwat MJ, Lam PYS, et al, Discovery of 1-(4-Methoxyphenyl)-7-oxo-6-(4-(2-oxopiperidin-1-yl)phenyl)-4,5,6,7-tetrahydro-1H-pyrazolo[3,4-c]pyridine-3-carboxamide (Apixaban, BMS-562247), a highly potent, selective, efficacious, and orally bioavailable inhibitor of blood coagulation factor Xa, J. Med. Chem., 50 (22), 5339-56, 2007];

(d) Betrixaban (N-(5-chloropyridin-2-yl)-2-[4-(N,N-dimethylcarbamimidoyl)benzamido]-5-methoxybenzamide) [See WFO Drug Information, Vol. 22, No. 3, 2008, page 226-227; Zhang P, Huang W, Zhu BY, et al., Discovery of betrixaban (PRT054021), N-(5-chloropyridin-2-yl)-2-(4-(N,N-dimethylcarbamimidoyl)benzamido)-5-methoxybenzamide, a highly potent, selective, and orally efficacious factor Xa inhibitor, Bioorg Med. Chem. Lett. 19 (8), 2179-85, 2009];

(e) AX-1826 [S. Takehana et al. Japanese Journal of Pharmacology 2000, 82 (Suppl. 1), 213P; T. Kayahara et al. Japanese Journal of Pharmacology 2000, 82 (Suppl. 1), 213P];

(f) HMR-2906, [XVIIth Congress of the International Society for Thrombosis and Haemostasis, Washington D. C., USA, 14-21 Aug 1999; Generating greater value from our products and pipeline. Aventis SA Company Presentation, 05 Feb 2004];

(g) Otamixaban (methyl (2R,3R)-2-(3-carbamimidoylbenzyl)-3-[[4-(1-oxidopyridin-4-yl)benzoyl]amino]butanoate) [See WFO Drug Information, Vol. 16, No. 3, 2002, page 257];

(h) BIBT-986 (prodrug: BIBT-1011) [American Chemical Society-226th National Meeting, New York City, NY, USA, 2003];

(i) DPC-602, [J. R. Pruitt et al. J. Med. Chem. 2003, 46, 5298-5313];

(j) LY517717 (N-[(1R)-2-[4-(1-methyl-4-piperidinyl)-1-piperazinyl]-2-oxo-1-phenylethyl]-1H-indole-6-carboxamide) [See S. Young, Medicinal Chemistry-12th RSC-SCI Symposium, 7-10 September 2003, Cambridge, UK; M. Wiley et al. 228th ACS National Meeting, Philadelphia, August 22-26, 2004, MEDI-252 & 254];

(k) $N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide or a pharmacologically acceptable salt thereof, or a hydrate thereof [see WO 2004/058715]; and

(l) $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide or a pharmacologically acceptable salt thereof, or a hydrate thereof [see WO 03/000657; WO 03/000680; and WO 03/016302].

The aforementioned activated blood coagulation factor X (FXa) inhibitor is more preferably a compound represented by the following formula (1) [hereinafter, also abbreviated to compound (1)]:

[Formula 1]

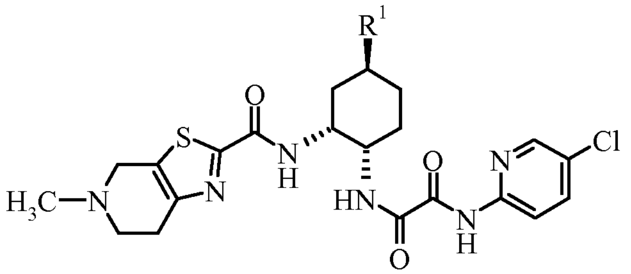

(1)

wherein $R^1$ represents an N,N-dimethylcarbamoyl group or a [1,3,4]oxadiazol-2-yl group. Compound (1) may be the free form (free base) or a pharmacologically acceptable salt thereof, or a hydrate thereof.

Examples of the salt of the compound represented by formula (1) include hydrochloride, sulfate, hydrobromide, hydroiodide, phosphate, nitrate, benzoate, methanesulfonate, 2-hydroxyethanesulfonate, p-toluenesulfonate, acetate, propionate, oxalate, malonate, succinate, glutarate, adipate, tartrate, maleate, fumarate, malate, and mandelate.

The salt of the compound represented by formula (1) is preferably maleate, hydrochloride, methanesulfonate, or p-toluenesulfonate, particularly preferably maleate or p-toluenesulfonate.

Preferable examples of the compound represented by formula (1) can include the following:

$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide monomaleate;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide;
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide hydrochloride; $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate; and
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate monohydrate.

Among these preferable compounds, particularly preferred are

$N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide monomaleate (1a); and
$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide mono-p-toluenesulfonate monohydrate (1b)

represented by the following formula (1a) [hereinafter, also abbreviated to compound (1a)] and formula (1b) [hereinafter, also abbreviated to compound (1b)], respectively:

[Formula 2]

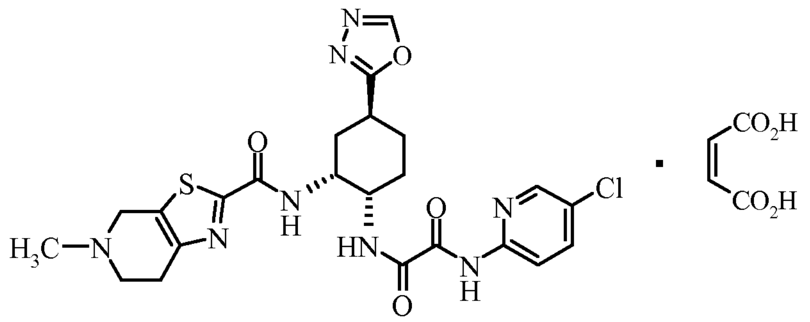

(1a)

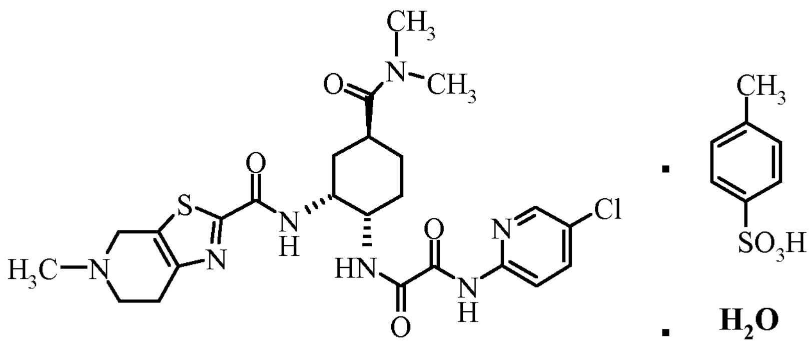

(1b)

The free base (free form) of compound (1) means the salt (acid-addition salt) and/or the hydrate formed with compound (1) except for "acid" in the acid-addition salt or "water" in the hydrate. For example, the free bases (free forms) of compound (1a) and compound (1b) mean $N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide (1a-1) and $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (1b-1)
represented by the following formula (1a-1) and formula (1b-1), respectively:

[Formula 3]

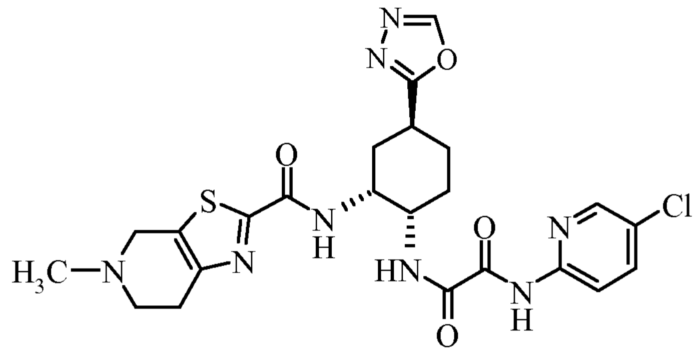

(1a-1)

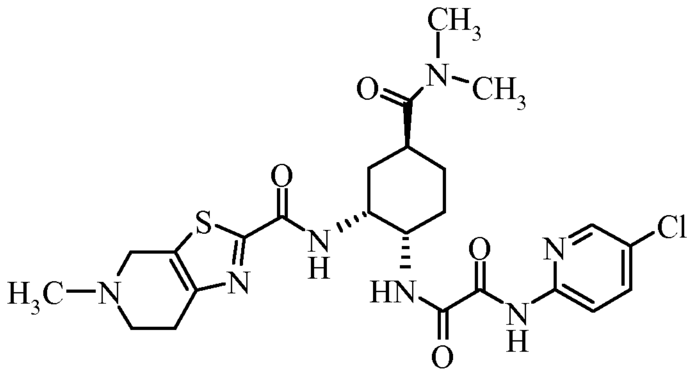

(1b-1)

These compounds (1) can be produced by a method described in above-mentioned documents (WO 2003-000657; WO 2003-000680; WO 2003-016302; and WO 2004-058715) or a method equivalent thereto.

Moreover, preferable examples of the pharmacologically active drug (A) of the present invention can include (±)-1-(carbazol-4-yloxy)-3-[[2-(o-methoxyphenoxy)ethyl]amino]-2-propanol (2) (CAS No.: 72956-09-3) represented by the following formula (2) [hereinafter, also abbreviated to compound (2)]:

[Formula 4]

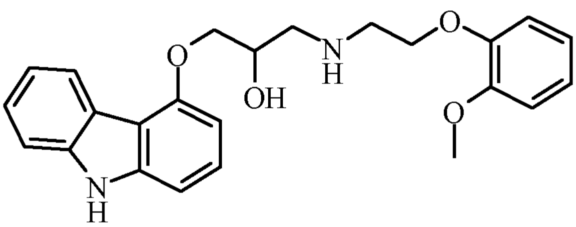

(2)

or a pharmacologically acceptable salt thereof, or a hydrate thereof.

In the present specification, the content of the "pharmacologically active drug (A)" in the "solid preparation" is preferably 0.1 to 60% by weight, more preferably 1 to 50% by weight, even more preferably 5 to 35% by weight, in terms of % by weight of the "pharmacologically active drug (A)", but is not limited to this range by any means.

In this context, "A to B% by weight" representing the content of each component in the present specification means the range "from A% by weight to B% by weight", unless otherwise specified.

In the present specification, commercially available Carbopol (Noveon/CBC) or the like can be used as the "carboxyvinyl polymer (B)", and various grades differing in viscosity are available. Preferable examples of the carboxyvinyl polymer can include Carbopol 974PNF.

In the present specification, the content of the carboxyvinyl polymer in the "solid preparation" is preferably 1 to 50% by weight, more preferably 5 to 30% by weight, further preferably 10 to 30% by weight.

In the present specification, the "povidone (polyvinylpyrrolidone) (C)" is a nonionic water-soluble polymer. The povidone means a linear polymer of 1-vinyl-2-pyrrolidone and does not encompass crospovidone, which is a cross-linked polymer of 1-vinyl-2-pyrrolidone. The povidone can be obtained with grades having various K values (characteristic values of viscosity correlating with molecular weights) from BASF Japan Ltd. or the like. Preferable examples thereof can include commercially available Kollidon 30 (BASF Japan Ltd.).

The content of the povidone according to the present invention in the "solid preparation" is 10 to 70% by weight.

Moreover, in the present specification, when the content of the carboxyvinyl polymer (B) is 10 to less than 15% by weight, the content of the povidone (C) is preferably 20 to 70% by weight.

In the present specification, examples of the sugar alcohol are mannitol, xylitol, or erythritol, particularly preferably xylitol.

In the present specification, the content of the sugar alcohol in the "solid preparation" is preferably up to 80% by weight, more preferably up to 60% by weight.

In the present specification, a possible swelling agent is xanthan gum or carmellose sodium. Carmellose sodium is particularly preferred. Commercially available carmellose sodium, for example, Sunrose (trade name; Nippon Paper Chemicals) can be used, and carmellose sodium having various viscosities and degrees of etherification is available.

The content of the swelling agent according to the present invention in the "solid preparation" is preferably 1 to 40% by weight, more preferably 5 to 15% by weight, even more preferably 7 to 15% by weight.

In addition to the pharmacologically active drug (A), the carboxyvinyl polymer (B), and the povidone (C), and the sugar alcohol and the swelling agent, the sustained-release solid preparation of the present invention may contain a disintegrant, a binder, a fluidizing agent, a lubricant, a coloring agent, a polishing agent, etc., so long as the effects of the present invention are not impaired.

Examples of the disintegrant include adipic acid, alginic acid, gelatinized starch, sodium carboxymethyl starch, hydrous silicon dioxide, calcium citrate, light anhydrous silicic acid, synthetic aluminum silicate, wheat starch, rice starch, calcium stearate, corn starch, tragacanth powder, potato starch, hydroxypropyl starch, pregelatinized starch, monosodium fumarate, anhydrous citric acid, calcium dihydrogenphosphate, croscarmellose sodium, crospovidone, carmellose and carmellose calcium.

Examples of the binder include maltose syrup powder, gum arabic, gum arabic powder, sodium alginate, propylene glycol alginate ester, hydrolyzed gelatin powder, hydrolyzed starch-light anhydrous silicic acid, fructose, carboxymethylethyl cellulose, hydrous silicon dioxide, agar powder, light anhydrous silicic acid, hydroxypropyl cellulose containing light anhydrous silicic acid, crystalline cellulose, synthetic aluminum silicate, copolydone, wheat flour, wheat starch, rice flour, rice starch, polyvinyl acetate resin, cellulose acetate phthalate, dioctyl sodium sulfosuccinate, dihydroxyaluminum aminoacetate, sodium potassium tartrate, water, sucrose fatty acid ester, purified gelatin, purified sucrose, gelatin, D-sorbitol, dextrin, starch, corn starch, tragacanth, tragacanth powder, lactose, concentrated glycerin, sucrose, potato starch, hydroxyethyl cellulose, hydroxyethyl methylcellulose, hydroxypropyl cellulose, hydroxypropyl starch, hydroxypropyl methylcellulose 2208, hydroxypropyl methylcellulose 2906, hydroxypropyl methylcellulose 2910, hydroxypropyl methylcellulose phthalate, vinylpyrrolidone-vinyl acetate copolymers, piperonyl butoxide, glucose, pregelatinized starch, fumaric acid, mixture of fumaric acid, stearic acid, polyvinyl acetal diethylamino acetate and hydroxypropyl methylcellulose 2910, pullulan, polyvinyl alcohol (completely saponified product), polyvinyl alcohol (partially saponified product), sodium polyphosphate, polyethylene glycol 4000, polyethylene glycol 6000, polyethylene glycol 20000, D-mannitol, and methylcellulose.

Examples of the fluidizing agent can include hydrous silicon dioxide, light anhydrous silicic acid, crystalline cellulose, synthetic aluminum silicate, titanium oxide, stearic acid, calcium stearate, magnesium stearate, calcium tertiary phosphate, talc, corn starch, and magnesium aluminometasilicate.

Examples of the lubricant include cocoa fat, carnauba wax, hydrous silicon dioxide, dry aluminum hydroxide gel, glycerin fatty acid ester, magnesium silicate, light anhydrous silicic acid, crystalline cellulose, hardened oil, synthetic aluminum silicate, white beeswax, magnesium oxide, sodium potassium tartrate, sucrose fatty acid ester, stearic acid, calcium stearate, magnesium stearate, stearyl alcohol, polyoxyl 40 stearate, cetanol, soybean hardened oil, gelatin, talc, magnesium carbonate, precipitated calcium carbonate, corn starch, potato starch, fumaric acid, stearyl sodium fumarate, polyethylene glycol 600, polyethylene glycol 4000, polyethylene glycol 6000, beeswax, magnesium metasilicate aluminate, sodium laurate, and magnesium sulfate.

Examples of the coloring agent can include yellow iron sesquioxide, iron sesquioxide, titanium oxide, orange essence, brown iron oxide, β-carotene, black iron oxide, food blue No. 1, food blue No. 2, food red No. 2, food red No. 3, food red No. 102, food yellow No. 4, and food yellow No. 5.

Examples of the polishing agent include carnauba wax, hardened oil, a polyvinyl acetate resin, white beeswax, titanium oxide, stearic acid, calcium stearate, polyoxyl 40 stearate, magnesium stearate, purified shellac, purified paraffin/carnauba wax mixture, cetanol, talc, colored silver foil, white shellac, paraffin, povidone (polyvinyl pyrrolidone), polyethylene glycol 1500, polyethylene glycol 4000, polyethylene glycol 6000, beeswax, glycerin monostearate, and rosin.

No particular limitation is imposed on the dosage form of the sustained-release solid preparation of the present invention, so long as the solid preparation thereof can be orally administered to a subject. However, a tablet and granules are preferred. The tablet is preferably a matrix tablet.

Moreover, the sustained-release solid preparation of the present invention may be produced through a widely known production method. In one exemplified procedure, the sustained-release solid preparation of the present invention is prepared through mixing the pharmacologically active drug (A), the carboxyvinyl polymer (B), and the povidone (C), and the sugar alcohol and swelling agent, and optional additives such as a disintegrant, a binder, a fluidizing agent, a lubricant, a coloring agent, and a polishing agent, and the mixture is processed through, for example, the method of producing solid preparations described in the general rules for preparations in the Japanese Pharmacopeia.

When the sustained-release solid preparation of the present invention is in the dosage form of granules, the components (A) to (C), and the sugar alcohol and swelling agent, and optional additives can be mixed in a granulation apparatus such as a fluidized-bed granulator and then granulated by wet granulation or the like to obtain granules. An additive such as a lubricant is added and mixed into the granules thus granulated to yield granules which are compressed into tablets. The granules can be compressed to yield tablets. The obtained tablets may be coated with a coating agent, if necessary, to yield film-coated tablets.

Alternatively, when the sustained-release solid preparation of the present invention is in the dosage form of a tablet, tablets may be produced directly through compression molding of a powder mixture containing the pharmacologically active drug (A), carboxyvinyl polymer (B), the povidone (C), and the sugar alcohol and swelling agent in combination, and acceptable additives for pharmaceuticals, preferably a powder mixture containing the pharmacologically active drug (A), the carboxyvinyl polymer (B), the povidone (C), the sugar alcohol, and the swelling agent in combination, and acceptable additives for pharmaceuticals. Moreover, the shape of the tablet is not particularly limited, preferably a lens, disc, round, oval, or a polygonal (e.g., triangle or rhombus) shape. Furthermore, the produced tablet may be further coated with a coating agent by means of a pan coater through spraying a suspension/solution of the coating agents onto the tablets.

The proportion of the pharmacologically active drug (A) contained in the pharmaceutical composition of the present invention is preferably in the range from 0.1% by weight to 60% by weight, more preferably 1 to 50% by weight, even more preferably 5 to 35% by weight, in terms of the free form of the pharmacologically active drug. Particularly, when the pharmaceutical composition of the present invention is in the dosage form of a tablet, the content of the pharmacologically active drug per tablet is in the range of 0.5 to 500 mg, preferably 1 to 100 mg, more preferably 5 to 75 mg, even more preferably 15 to 60 mg, in terms of the free form of the pharmacologically active drug.

Moreover, in an evaluation method for the hydration rate of the sustained-release solid preparation of the present invention, each tablet was left standing at 37 ± 0.5°C for 2 hours in 0.01 N hydrochloric acid. Then, the volume of the unhydrated portion was determined, and the hydration rate was calculated according to an equation represented by the following equation 1:

[Expression 1]

$$\text{Hydration rate}(\%) = \frac{\text{Initial volume of tablet} - \text{Volume of unhydrated portion in tablet}}{\text{Initial volume of tablet}} \times 100 \qquad \text{(Equation 1)}$$

The hydration rate is preferably 60% or more, more preferably 90% or more.

In an evaluation method for the swelling rate of the pharmaceutical composition of the present invention, the swelling rate can be calculated according to an equation represented by the following equation 2:

[Expression 2]

$$\text{Swelling rate}(\%) = \frac{\text{Volume of tablet after 2 hours}}{\text{Initial volume of tablet}} \times 100 \qquad \text{(Equation 2)}$$

The swelling rate is preferably 150% or more, more preferably 180% or more, particularly preferably 200% or more.

Hereinafter, another embodiment of the present invention will be described.

The sustained-release solid preparation of the present invention contains (A) a pharmacologically active drug, (B) a carboxyvinyl polymer, (C) povidone, (D) carmellose sodium or xanthan gum, and (E) a sugar alcohol.

No particular limitation is imposed on the "pharmacologically active drug" used as the component (A) in the preparation of the present invention, and the drug may be a prodrug that can be converted to the pharmacologically active drug *in vivo.* Preferable examples of the component (A) of the present invention can include those described above, but are not limited to these by any means. The content of the component (A) in the preparation of the present invention is preferably 0.1 to 60% by weight, more preferably 1 to 50% by weight, even more preferably 5 to 35% by weight, but is not limited to this range by any means.

The "carboxyvinyl polymer" used as the component (B) in the preparation of the present invention can be any of those described above, and commercially available Carbopol 974PNF is preferred. The content of the component (B) in the preparation of the present invention is preferably 1 to 50% by weight, more preferably 5 to 30% by weight, even more preferably 10 to 30% by weight.

The "povidone" used as the component (C) in the preparation of the present invention means those described above, and commercially available Kollidon 30 (BASF Japan Ltd.) is preferred. The content of the component (C) in the preparation of the present invention is 10 to 70% by weight.

Moreover, when the content of the component (B) is 10 to less than 15% by weight, the content of the component (C) is preferably 20 to 70% by weight.

The component (D) used in the preparation of the present invention is carmellose sodium or xanthan gum and is preferably carmellose sodium. The content of the component (D) in the preparation of the present invention is preferably 5 to 15% by weight, more preferably 7 to 15% by weight.

In addition to the components (A) to (D), the preparation of the present invention contains a sugar alcohol (E). Examples of the sugar alcohol include those described above. Mannitol, xylitol, or erythritol is preferred with xylitol being more preferred. The content of the sugar alcohol in the preparation of the present invention is preferably 10 to 40% by weight.

The preparation of the present invention may further contain additives such as the binder, the fluidizing agent, the lubricant, the coloring agent, the polishing agent, etc., so long as the effects of the present invention are not impaired.

No particular limitation is imposed on the dosage form of the preparation of the present invention, so long as the preparation can be orally administered to a subject. However, a tablet is preferred. The tablet is preferably a matrix tablet.

The preparation of the present invention is obtained by mixing of the components (A) to (D) and the sugar alcohol followed by compression into tablets or by mixing and granulation of the components (A) to (D) and the sugar alcohol followed by compression into tablets. The mixing, granulation, and compression into tablets can be performed using methods well known in the art.

No particular limitation is imposed on the shape of the tablet. However, a lens, disc, round, oval, or polygonal (triangle or rhombus) shape is preferred. The preparation of the present invention may be film-coated, if necessary, with a coating agent. The mixing, granulation, compression, and coating can be performed using methods well known in the art.

When the preparation of the present invention contains additional additives, these additives may be added thereto in any of mixing, granulation, compression, and coating steps.

As described in Advantageous Effects of the Invention above, the preparation of the present invention thus obtained has a favorable hydration rate and swelling rate in an acidic solution and has a favorable tablet strength that prevents dose dumping. In addition, the preparation of the present invention has favorable dissolution properties in a neutral solution. Thus, the preparation is effective for maintenance of prolonged dissolution of the pharmacologically active drug contained therein from the duodenum through the small intestine to the lower gastrointestinal tract.

Moreover, in the evaluation methods for the hydration rate and swelling rate of the preparation of the present invention, these rates can be calculated according to above equations 1 and 2. The hydration rate is more preferably 90% or more. The swelling rate is preferably 150% or more, more preferably 180% or more, particularly preferably 200% or more.

Examples

Next, the present invention will be described in detail with reference to Examples. However, the present invention is not intended to be limited to these by any means.

Tests on dissolution properties in an acidic or neutral solution were conducted as follows:

(Dissolution test in acidic solution)

The dissolution test was conducted by the paddle method at rotation rates of 50 rpm and 200 rpm at 37 ± 0.5°C in 0.01 N hydrochloric acid (900 mL), and the time-dependent average percentage dissolution of a drug in the dissolution medium was calculated. The average percentage dissolution at each rotation rate, the difference in the average percentage dissolution of the drug (value at the rotation rate of 200 rpm in the paddle method - value at the rotation rate of 50 rpm in the paddle method: $D_{2h,200rpm}$ - $D_{2h,50rpm}$), and the average percentage dissolution ratio (value at the rotation rate of 200 rpm in the paddle method / value at the rotation rate of 50 rpm in the paddle method: $D_{2h,200rpm}$ / $D_{2h,50rpm}$) derived from the dissolution test for 2 hours were calculated.

(Dissolution test in neutral solution)

The dissolution test was conducted by the paddle method at a rotation rate of 50 rpm at 37 ± 0.5°C in phosphate buffer (pH 6.8, 900 mL), and the time-dependent average percentage dissolution of a drug in the dissolution medium was calculated.

The hydration rate and swelling rate of each tablet were measured as follows:

(Evaluation of hydration rate of tablet)

In the evaluation method for the hydration rate, each tablet was left standing at 37 ± 0.5°C for 2 hours in 0.01 N hydrochloric acid. Then, the hydration rate was calculated according to above equation 1.

(Evaluation of swelling rate)

Each tablet was left standing for 2 hours in 0.01 N hydrochloric acid (1000 mL) and then taken out thereof. The swelling rate was calculated according to above equation 2 from the volume of the tablet thus left standing.

(Example 1)

(Production of tablet)

Ingredients described in Table 1 were mixed using an agate mortar, and 200 mg of this powder mixture was compressed into tablets [tablet diameter: 8.0 mm (flat tablet)] using a single-punch tableting machine (N-30E, Okada Seiko Co., Ltd.) and used as samples.

[Table 1]

| | Content (mg) | | | |
|---|---|---|---|---|
| | Comparative Example 1 | Comparative Example 2 | Comparative Example 3 | Formulation 1** |
| Compound (1a) | 36.4 | 36.4 | 36.4 | 36.4 |
| Carboxyvinyl polymer | 50.0 | 50.0 | 50.0 | 50.0 |
| Xylitol | 113.6 | - | - | - |
| Polyethylene glycol 6000 | - | 113.6 | - | - |
| Polyoxyethylene (160) polyoxypropylene (30) glycol | - | - | 113.6 | - |
| Povidone | - | - | - | 113.6 |
| Total | 200.0 | 200.0 | 200.0 | 200.0 |
| Diameter (mm) | 8.0 | 8.0 | 8.0 | 8.0 |

[Table 2]

| | Comparative Example 1 | Comparative Example 2 | Comparative Example 3 | Formulation 1** |
|---|---|---|---|---|
| Hydration rate (%) | >90 | >90 | >90 | >90 |
| **comparative example | | | | |

<Evaluation results>

As shown in Table 2, all the tablets of these formulations had a hydration rate of 90% or more, demonstrating that most internal parts of the tablets were hydrated.

(Evaluation of dissolution properties in acidic solution of formulations in Table 1)

The tablets of each formulation shown in Table 1 were tested for their dissolution properties in an acidic solution. The results are shown in Table 3 and Figures 1 to 4.

[Table 3]

| | | Comparative Example 1 | Comparative Example 2 | Comparative Example 3 | Formulation 1** |
|---|---|---|---|---|---|
| $D_{2hr}$ | 200rpm/50rpm (ratio) | 3.7 | 2.6 | 2.7 | 1.0 |
| | 200rpm - 50rpm (%) | 63.2 | 39.4 | 42.2 | 0.2 |
| **comparative example | | | | | |

<Evaluation results>

The tablets of formulation 1 were confirmed to suppress drug release and be less influenced by the paddle rotation rate, compared with the tablets having formulations of Comparative Examples 1 to 3, demonstrating that the simultaneous addition of the carboxyvinyl polymer and povidone was effective for maintenance of tablet strength in an acidic solution.

The tablets of formulation 1 were tested for their dissolution properties in a neutral solution. The results are shown in Figure 5.

<Evaluation results>

As shown in Figure 5, the tablets of formulation 1 exhibited prolonged dissolution properties in the neutral solution.

(Example 2)

Ingredients of each formulation shown in Table 4 were mixed using an agate mortar, and 200 mg of this powder mixture was compressed into tablets [tablet diameter: 8.0 mmϕ (6.5R)] using a single-punch tableting machine (N-30E, Okada Seiko Co., Ltd.) and used as samples.

The obtained samples were subjected to the dissolution test in an acidic solution, and the results are shown in Figures 6 and 7 and Table 5.

[Table 4]

| | Content (mg) | |
|---|---|---|
| | Formulation 2a** (Povidone 5%) | Formulation 2b** (Povidone 10%) |
| Compound (1a) | 36.4 | 36.4 |
| Carboxyvinyl polymer | 50.0 | 50.0 |
| Xylitol | 103.6 | 93.6 |
| Povidone | 10.0 | 20.0 |
| Total | 200.0 | 200.0 |
| Diameter (mm) | 8.0 | 8.0 |

[Table 5]

| | | Formulation 2a** | Formulation 2b** |
|---|---|---|---|
| $D_{2hr}$ | 200rpm/50 rpm (ratio) | 3.8 | 1.3 |
| | 200rpm-50rpm (%) | 66.9 | 7.0 |
| **comparative example | | | |

<Test results>

As shown in Figures 6 and 7 and Table 5, the influence of the paddle rotation rate on dissolution in the acidic solution was small in the tablets of formulation 2b, but relatively large in the tablets of formulation 2a. This result demonstrated that the addition of 10% by weight or more of povidone produced a favorable tablet strength.

(Example 3)

Ingredients of each formulation shown in Table 6 were mixed using an agate mortar, and 200 mg of this powder mixture was compressed into tablets [tablet diameter: 8.0 mmϕ (6.5R)] using a single-punch tableting machine (N-30E, Okada Seiko Co., Ltd.) and used as samples.

The tablets of formulations 1 and 3 containing 25% and 15%, respectively, of a carboxyvinyl polymer were subjected to the dissolution test in an acidic solution, and the results are shown in Figures 4 and 8 and Table 7.

[Table 6]

| | Content (mg) | |
|---|---|---|
| | Formulation 1** (Carboxyvinyl polymer: 25%) | Formulation 3** (Carboxyvinyl polymer: 15%) |
| Compound (1a) | 36.4 | 36.4 |
| Carboxyvinyl polymer | 50.0 | 30.0 |
| Povidone | 113.6 | 133.6 |
| Total | 200.0 | 200.0 |
| Diameter (mm) | 8.0 | 8.0 |

[Table 7]

| | | Formulation 1** (Carboxyvinyl polymer: 25%) | Formulation 3** (Carboxyvinyl polymer: 15%) |
|---|---|---|---|
| $D_{2hr}$ | 200rpm/50rpm (ratio) | 1.0 | 1.3 |
| | 200rpm-50rpm (%) | 0.2 | 3.0 |
| **comparative example | | | |

<Evaluation results>

As shown in the dissolution behaviors of Figures 4 and 8 and Table 7, both the tablets of formulations 1 and 3 supplemented with 25% and 15%, respectively, of the carboxyvinyl polymer had little effect from the paddle rotation rate in the acidic solution.

(Example 4)

Ingredients of each formulation shown in Table 8 were mixed using an agate mortar and compressed using a single-punch tableting machine (N-30E) to produce tablets [tablet diameter: 10.0 mmϕ (8R)].

Results of testing dissolution properties in acidic and neutral solutions and results of determining the hydration rate and swelling rate of each formulation are shown in Table 9 and Figures 9 to 12.

[Table 8]

| | Content (mg) | | | |
|---|---|---|---|---|
| | Formulation 4** | Formulation 5 | Formulation 6 | Formulation 7** |
| Compound (1a) | 36.4 | 36.4 | 36.4 | 36.4 |
| Carboxyvinyl polymer | 35.0 | 40.0 | 40.0 | 40.0 |
| Xylitol | 183.6 | 148.6 | 148.6 | 148.6 |
| Povidone | 105.0 | 105.0 | 105.0 | 105.0 |
| Carmellose sodium | - | 30.0 | - | - |
| Xanthan gum | - | - | 30.0 | - |
| Sodium carboxymethyl starch | - | - | - | 30.0 |
| Total | 360.0 | 360.0 | 360.0 | 360.0 |
| Diameter (mm) | 10.0 | 10.0 | 10.0 | 10.0 |

[Table 9]

| | | Formulation 4** | Formulation 5 | Formulation 6 | Formulation 7** |
|---|---|---|---|---|---|
| $D_{2hr}$ in 0.01 N hydrochloric acid | 200 rpm/50 rpm (ratio) | 1.1 | 1.1 | 1.5 | 1.1 |
| | 200 rpm-50 rpm (%) | 2.7 | 2.9 | 9.1 | 2.1 |
| Swelling rate (%) | | 109 | 243 | 253 | 161 |
| Hydration rate (%) | | > 95 | > 95 | > 95 | > 95 |
| **comparative example | | | | | |

<Test results>

As shown in Figures 9 to 12, all the tablets of these formulations exhibited prolonged dissolution behaviors in the neutral region. Moreover, as shown in Table 9, the tablets of formulation 4 were rapidly hydrated, but hardly swelled. The swelling rate thereof after 2 hours was 109%. By contrast, the tablets of formulations 5 to 7 supplemented with carmellose sodium, xanthan gum, or sodium carboxymethyl starch as a swelling agent were confirmed to be rapidly hydrated and swelled in 2 hours by about 1.6 times to about 2.5 times compared with their initial sizes. The tablets of formulations 5 to 7 had a favorable hydration rate and swelling rate in the acidic solution and had little effect from the paddle rotation rate in the acidic solution. Particularly, the tablets of formulation 5 supplemented with carmellose sodium were excellent in hydration rate, swelling rate, and tablet strength in the acidic solution.

(Example 5)

The tablets of formulations 4 and 5 shown in Table 8 were evaluated for their absorption properties in the large intestine of each fasted dog. In this test, the total amount of each formulation shown in Table 8 was reduced to approximately 2/3, and the evaluation was conducted using 240 mg in total of each preparation having a compound (1a-1) content of 20 mg (formulations 4' and 5').

For the administration of each preparation into the large intestine, one tablet dipped for 2 hours in 0.01 N hydrochloric acid was loaded in an endoscope for animals (Olympus Corp.) and administered from the anus to a site about 30 cm therefrom while the canine large intestine was observed with the endoscope. Before the administration, the amount of a portion of the drug dissolved from the tablet into 0.01 N hydrochloric acid was measured, and the plasma concentration of the drug was calculated such that the dose was 20 mg of compound (1a-1)/head. Change in the plasma

concentration of the drug in this test is shown in Figure 13.

<Evaluation results>

As shown in Figure 13, the drug of formulation 5' containing carmellose sodium as a swelling agent exhibited larger absorption properties in the large intestine than that of formulation 4' free from carmellose sodium, demonstrating that water swelling effect contributed to improvement in absorption properties. This result demonstrated that the presence of a swelling agent was important for improvement in prolonged absorption properties in the lower gastrointestinal tract.

(Example 6)

Compound (1b), a carboxyvinyl polymer, carmellose sodium, povidone, polyethylene glycol 6000, xylitol, and sodium stearyl fumarate were placed at weight ratios shown in Table 10 in a mortar and mixed for 3 minutes to yield a powder mixture. The predetermined amount per tablet shown in Table 10 was weighed for this powder mixture and compressed into tablets (die: 10 mmϕ, flat) using a single-punch tableting machine (N-30E, Okada Seiko Co., Ltd.).

The tablets of formulation 8 and Comparative Examples 4 and 5 were tested for their dissolution properties in an acidic aqueous solution, and the results are shown in Table 11.

The hydration rates and swelling rates of the tablets of formulations 8, 9, and 10 were determined, and the results are shown in Table 12.

[Table 10]

| | Content (mg) | | | | |
|---|---|---|---|---|---|
| | Comparative Example 4 | Comparative Example 5 | Formulation 8 | Formulation 9** | Formulation 10** |
| Compound (1b) | 80.8 | 80.8 | 80.8 | 80.8 | 80.8 |
| Carboxyvinyl polymer | 60.0 | - | 60.0 | 60.0 | 60.0 |
| Carmellose sodium | 35.0 | 35.0 | 35.0 | - | 35.0 |
| Povidone | - | 105.0 | 105.0 | 105.0 | 105.0 |
| Polyethylene glycol6000 | 105.0 | - | - | - | - |
| Xylitol | 64.8 | 64.8 | 64.8 | 64.8 | - |
| Sodium stearyl fumarate | 14.4 | 14.4 | 14.4 | 14.4 | 14.4 |
| Total | 360.0 | 300.0 | 360.0 | 325.0 | 295.2 |
| **comparative example | | | | | |

[Table 11]

| | | Comparative Example 4 | Comparative Example 5 | Formulation 8 |
|---|---|---|---|---|
| $D_{2hr}$ in 0.01 N hydrochloric acid | 200 rpm/50 rpm (ratio) | 1.9 | 3.5 | 1.2 |
| | 200 rpm-50 rpm (%) | 11.9 | 70.6 | 2.7 |

[Table 12]

| | Formulation 8 | Formulation 9** | Formulation 10** |
|---|---|---|---|
| Swelling rate | 196 | 116 | 163 |
| Hydration rate | 100 | 94.5 | 75.9 |
| **comparative example | | | |

<Evaluation results>

In Comparative Examples 4 and 5, the ratio (dissolution rate at a paddle rotation rate of 50 rpm / dissolution rate at a paddle rotation rate of 200 rpm) was 1.9 and 3.5, respectively. By contrast, in formulation 8, this ratio was 1.2, showing the small influence of the rotation rate. Moreover, the tablets of formulations 9 and 10 had a swelling rate of 116% and 163%, respectively, and a hydration rate of 94.5% and 75.9%, respectively. By contrast, the tablets of formulation 8 had a swelling rate of 196% and a hydration rate of 100%, demonstrating that the addition of carmellose sodium as a swelling agent was effective. Moreover, the tablets of formulation 10 free from the sugar alcohol xylitol had a lower hydration rate than that of the tablets of formulations 8 and 9 containing xylitol.

(Example 7)

Since the tablets of formulation 5 were hardly influenced by the paddle rotation rate in the acidic solution and exhibited prolonged dissolution properties in the neutral solution and a favorable swelling rate and hydration rate, a preparation having a formulation shown in Table 13 was produced. Specifically, drug (1a), a carboxyvinyl polymer, xylitol, povidone (a portion), carmellose sodium, and an additional additive were added at ratios shown in Table 13 to a fluidized-bed granulator and mixed. Then, a povidone solution (remaining portion) was sprayed thereon as a binding solution for wet granulation. The obtained granules were dried, and sodium stearyl fumarate was then added to the granules thus granulated, and mixed using a V-shaped mixer to yield granules which were compressed into tablets. The granules were compressed (die: 10 mmϕ) using a rotary tableting machine to yield plain tablets. An aqueous dispersion of a coating base composed of hypromellose 2910, talc, titanium oxide, and polyethylene glycol was sprayed onto the plain tablets using a pan coater to yield film-coated tablets.

The obtained preparation was test for its dissolution properties in an acidic solution and determined for its hydration rate and swelling rate, and the results are shown in Tables 14 and 15.

[Table 13]

| | Content (mg) | | |
|---|---|---|---|
| | Formulation 11a | Formulation 11b | Formulation 11c |
| Compound (1a) | 36.4 | 36.4 | 36.4 |
| Carboxyvinyl polymer | 39.0 | 100.0 | 60.0 |
| Xylitol | 135.2 | 54.2 | 109.2 |
| Povidone | 105.0 | 105.0 | 105.0 |
| Carmellose sodium | 30.0 | 50.0 | 35.0 |
| Sodium stearyl fumarate | 14.4 | 14.4 | 14.4 |
| Total | 360.0 | 360.0 | 360.0 |
| Diameter (mm) | 10.0 | 10.0 | 10.0 |

[Table 14]

| | | Formulation 11a | Formulation 11b | Formulation 11c |
|---|---|---|---|---|
| $D_{2hr}$ in 0.01 N hydrochloric acid | 200 rpm/50 rpm (ratio) | 1.5 | 1.5 | 1.5 |
| | 200 rpm-50 rpm (%) | 12.9 | 2.8 | 10.8 |

[Table 15]

| | Formulation 11a | Formulation 11b | Formulation 11c |
|---|---|---|---|
| Swelling rate (%) | 273 | 242 | 263 |
| Hydration rate (%) | 100 | 100 | 100 |

<Test results>

As is evident from the evaluation results, the film-coated granulated preparations of formulations 11a to 11c had an average percentage dissolution in the acidic solution almost equivalent to the effect of the tablets. Moreover, the preparations of formulations 11a to 11c exhibited, as in the tablets of Examples 4 and 5, lower Cmax and a higher blood concentration after 24 hours than those of an administered aqueous solution having the same amount of the drug in an administration test in humans, demonstrating that these preparations exhibited favorable profiles as sustained-release preparations.

(Preparation Example 1)

A preparation having a formulation shown in Table 16 was produced using compound (2) as a drug.

[Table 16]

| | Content (mg) |
|---|---|
| | Formulation 12 |
| Compound (2) | 10.0 |
| Carboxyvinyl polymer | 35.0 |
| Xylitol | 45.0 |
| Povidone | 50.0 |
| Carmellose sodium | 15.0 |
| Total | 155.0 |

<Test results>

The bioavailability (BA) of the preparation of formulation 12 in dogs exhibited performance as high as 1.54 times that of the existing sustained-release preparation (Coreg CR) containing compound (2), and its time to the maximum plasma concentration (Tmax) was prolonged by 2 times or more compared with the existing sustained-release preparation, demonstrating that the preparation of formulation 12 had preferable profiles as a sustained-release preparation.

Industrial Applicability

The present invention can be used in the production of a sustained-release solid preparation for oral administration containing a pharmacologically active drug, for example, compound (1).

## Claims

**1.** A sustained-release solid preparation containing

(A) a pharmacologically active drug,
(B) a carboxyvinyl polymer,
(C) povidone, and
(D) carmellose sodium or xanthan gum, and
(E) a sugar alcohol,

wherein the content of the component (C) in the preparation is 10 to 70% by weight.

2. The preparation according to claim 1, wherein the component (D) in the preparation is carmellose sodium.

3. The preparation according to claim 1 or 2, wherein the content of the component (A) in the preparation is 5 to 35% by weight.

4. The preparation according to any one of claims 1 to 3, wherein the content of the component (B) in the preparation is 5 to 30% by weight.

5. The preparation according to any one of claims 1 to 4, wherein the content of the component (D) in the preparation is 5 to 15% by weight.

6. The preparation according to any one of claims 1 to 5, wherein the content of the sugar alcohol in the preparation is 10 to 40% by weight.

7. The preparation according to any one of claims 1 to 6, wherein the sugar alcohol is mannitol, xylitol, or erythritol.

8. The preparation according to any one of claims 1 to 6, wherein the sugar alcohol is xylitol.

9. The preparation according to any one of claims 1 to 8, wherein the component (A) is a basic drug.

10. The preparation according to any one of claims 1 to 8, wherein the component (A) is a compound selected from the group consisting of

    (±)-1-(carbazol-4-yloxy)-3-[[2-(o-methoxyphenoxy)ethyl]amino]-2-propanol,
    $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide, and
    $N^1$-(5-chloropyridin-2-yl)-$N^2$-[(1S,2R,4S)-2-{[(5-methyl-4,5,6,7-tetrahydrothiazolo[5,4-c]pyridin-2-yl)carbonyl]amino}-4-([1,3,4]oxadiazol-2-yl)cyclohexyl]ethanediamide

    or a pharmacologically acceptable salt thereof, or a hydrate thereof.

11. The preparation according to any one of claims 1 to 10, wherein the dosage form is a tablet.

**Patentansprüche**

1. Feststoffpräparat mit verzögerter Freisetzung, enthaltend

    (A) ein pharmakologisch aktives Medikament,
    (B) ein Carboxyvinylpolymer,
    (C) Povidon und
    (D) Carmellose-Natrium- oder Xantan-Gummi und
    (E) einen Zuckeralkohol,

    wobei der Gehalt der Komponente (C) in dem Präparat 10 bis 70 Gew.-% beträgt.

2. Präparat nach Anspruch 1, wobei die Komponente (D) in dem Präparat Carmellose-Natrium ist.

3. Präparat nach Anspruch 1 oder 2, wobei der Gehalt der Komponente (A) in dem Präparat 5 bis 35 Gew.-% beträgt.

4. Präparat nach einem der Ansprüche 1 bis 3, wobei der Gehalt der Komponente (B) in dem Präparat 5 bis 30 Gew.-% beträgt.

5. Präparat nach einem der Ansprüche 1 bis 4, wobei der Gehalt der Komponente (D) in dem Präparat 5 bis 15 Gew.-% beträgt.

6. Präparat nach einem der Ansprüche 1 bis 5, wobei der Gehalt des Zuckeralkohols in dem Präparat 10 bis 40 Gew.-% beträgt.

7. Präparat nach einem der Ansprüche 1 bis 6, wobei der Zuckeralkohol Mannit, Xylit oder Erythrit ist.

8. Präparat nach einem der Ansprüche 1 bis 6, wobei der Zuckeralkohol Xylit ist.

9. Präparat nach einem der Ansprüche 1 bis 8, wobei die Komponente (A) ein Basismedikament ist.

10. Präparat nach einem der Ansprüche 1 bis 8, wobei die Komponente (A) eine Verbindung ausgewählt aus der Gruppe bestehend aus

(±)-1-(Carbazol-4-yloxy)-3-[[2-(o-methoxyphenoxy)ethyl]amino]-2-propanol,
$N^1$-(5-Chlorpyridin-2-yl)-$N^2$-((1S,2R,4S)-4-[(dimethylamino)carbonyl]-2-{[(5-methyl-4,5,6,7-tetrahydrothiazol[5,4-c]pyridin-2-yl)carbonyl]amino}cyclohexyl)ethandiamid und
$N^1$-(5-Chlorpyridin-2-yl)-$N^2$-[(1S,2R,4S)-2-{[(5-methyl-4,5,6,7-tetrahydrothiazol[5,4-c]pyridin-2-yl)carbonyl]amino}-4-([1,3,4]oxadiazol-2-yl)cyclohexyl]ethandiamid

oder ein pharmakologisch akzeptables Salz davon oder ein Hydrat davon ist.

11. Präparat nach einem der Ansprüche 1 bis 10, wobei die Darreichungsform eine Tablette ist.

**Revendications**

1. Préparation solide à libération prolongée contenant

(A) un médicament pharmacologiquement actif,
(B) un polymère de carboxyvinyle,
(C) de la povidone, et
(D) de la carmellose sodique ou de la gomme de xanthane, et
(E) un alcool de sucre,

dans laquelle la teneur du composant (C) dans la préparation est de 10 à 70 % en poids.

2. Préparation selon la revendication 1, dans laquelle le composant (D) dans la préparation est la carmellose sodique.

3. Préparation selon la revendication 1 ou 2, dans laquelle la teneur du composant (A) dans la préparation est de 5 à 35 % en poids.

4. Préparation selon l’une quelconque des revendications 1 à 3, dans laquelle la teneur du composant (B) dans la préparation est de 5 à 30 % en poids.

5. Préparation selon l’une quelconque des revendications 1 à 4, dans laquelle la teneur du composant (D) dans la préparation est de 5 à 15 % en poids.

6. Préparation selon l’une quelconque des revendications 1 à 5, dans laquelle la teneur de l’alcool de sucre dans la préparation est de 10 à 40 % en poids.

7. Préparation selon l’une quelconque des revendications 1 à 6, dans laquelle l’alcool de sucre est le mannitol, le xylitol, ou l’érythritol.

8. Préparation selon l’une quelconque des revendications 1 à 6, dans laquelle l’alcool de sucre est le xylitol.

9. Préparation selon l’une quelconque des revendications 1 à 8, dans laquelle le composant (A) est un médicament basique.

10. Préparation selon l’une quelconque des revendications 1 à 8, dans laquelle le composant (A) est un composé

sélectionné dans le groupe constitué

du (±)-1-(carbazol-4-yloxy)-3-[[2-(o-méthoxyphénoxy)éthyl]amino]-2-propanol,
du $N^1$-(5-chloropyridin-2-yl)-$N^2$-((1S,2R,4S)-4-[(diméthylamino)carbonyl]-2-{[(5-méthyl-4,5,6,7-tétrahydrothiazolo[5,4-c]pyridin-2-yl)carbonyl]amino}cyclohexyl)éthanediamide, et
du $N^1$-(5-chloropyridin-2-yl)-$N^2$-[(1S,2R,4S)-2-{[(5-méthyl-4,5,6,7-tétrahydrothiazolo[5,4-c]pyridin-2-yl)carbonyl]amino}-4-([1,3,4]oxadiazol-2-yl)cyclohexyl]éthanediamide

ou d'un sel pharmacologiquement acceptable de celui-ci, ou d'un hydrate de celui-ci.

**11.** Préparation selon l'une quelconque des revendications 1 à 10, dans laquelle la forme posologique est un comprimé.

[Figure 1]

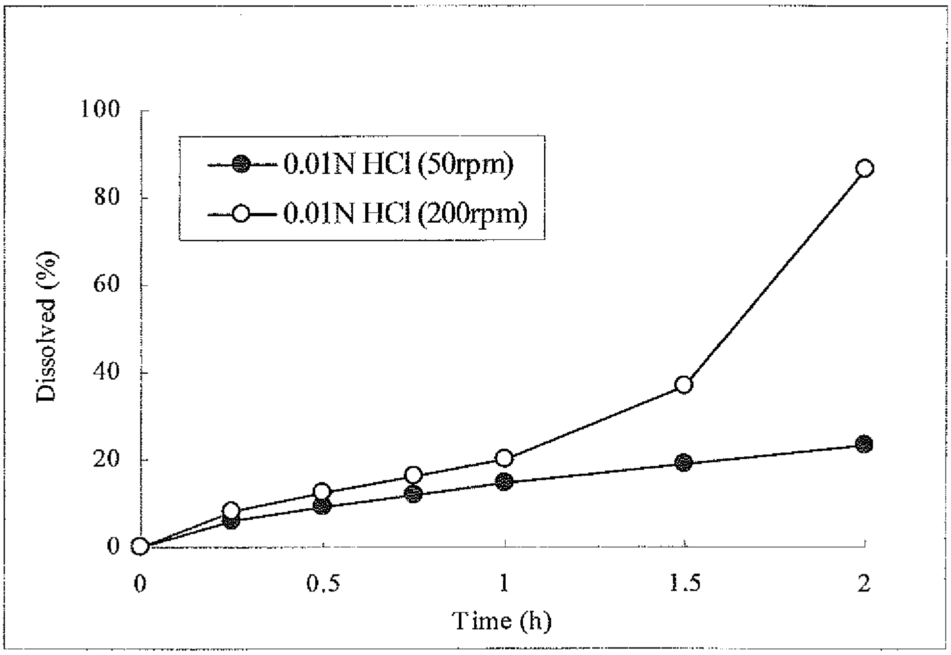


[Figure 2]

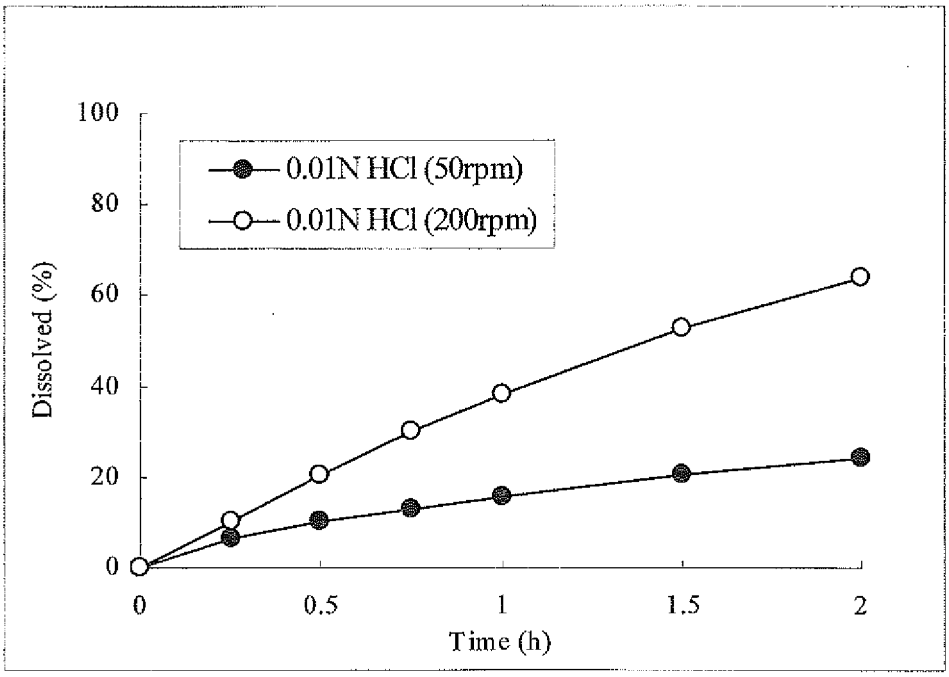

[Figure 3]

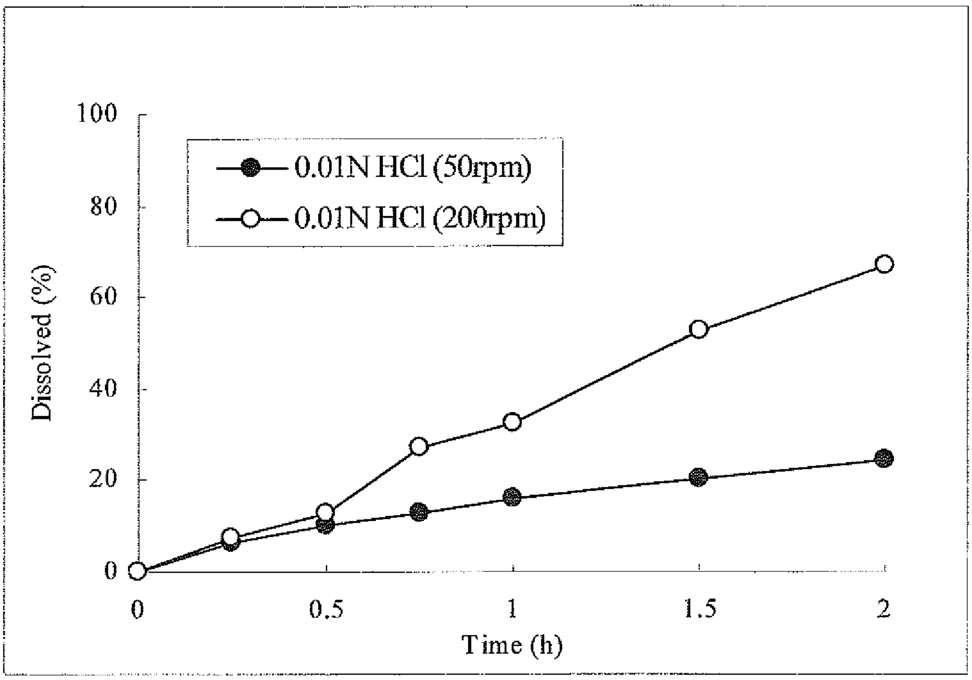


[Figure 4]

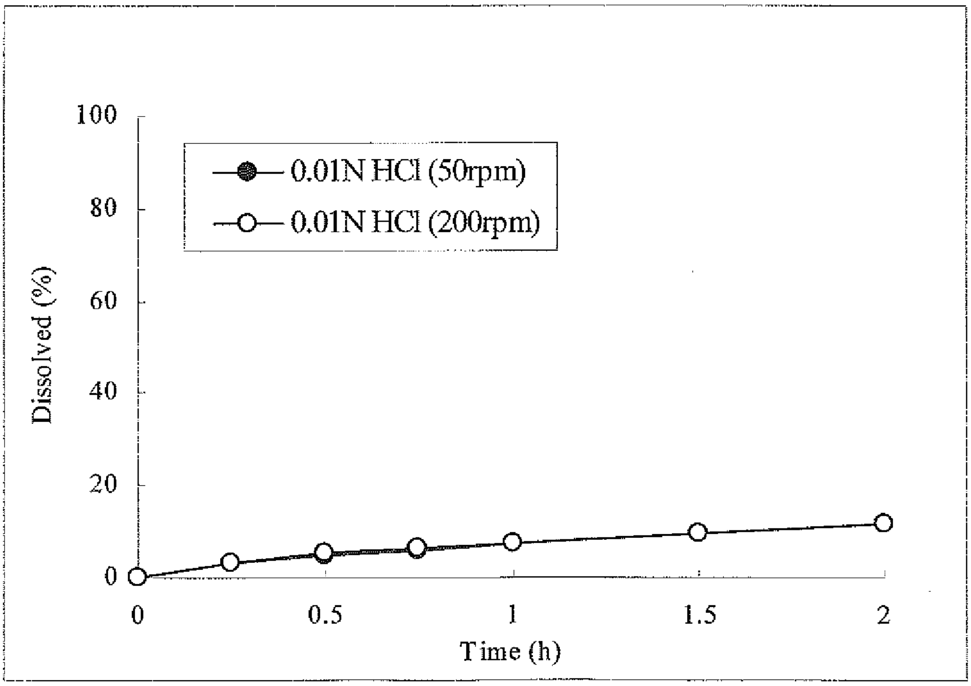

[Figure 5]

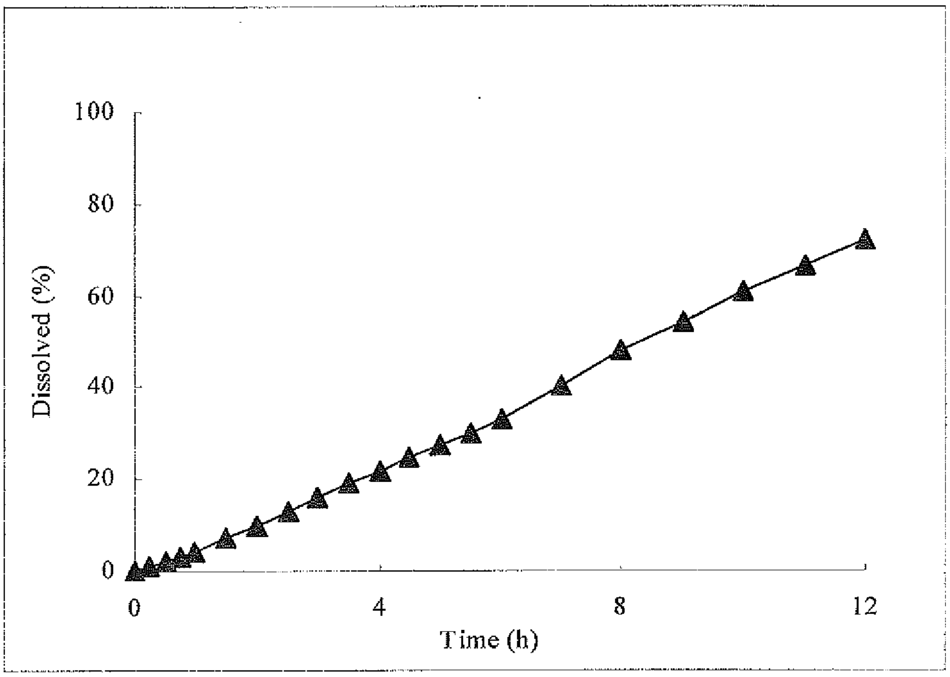


[Figure 6]

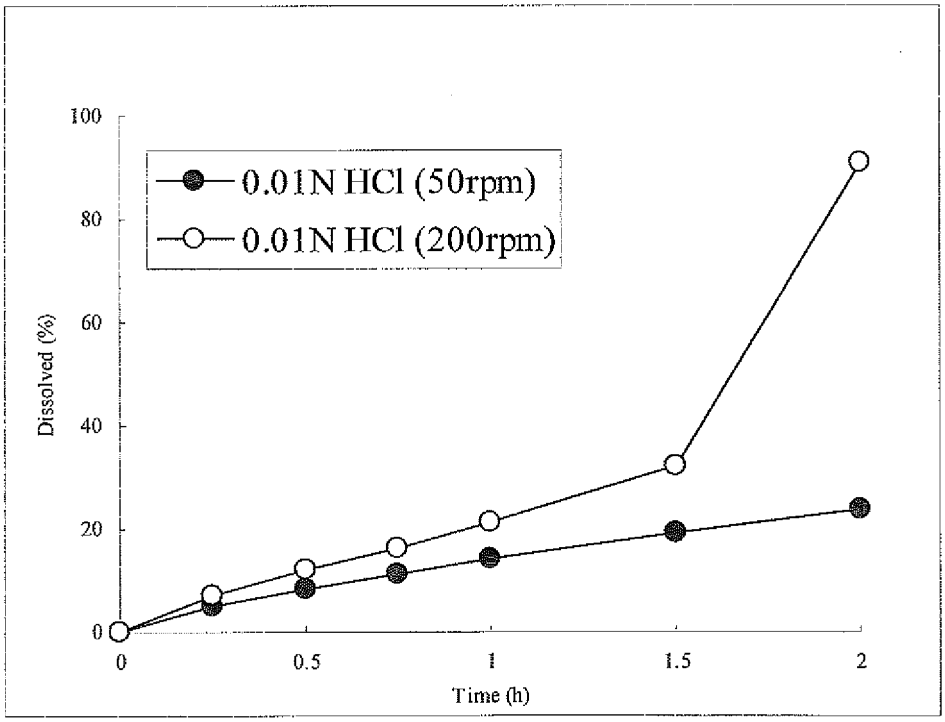

[Figure 7]

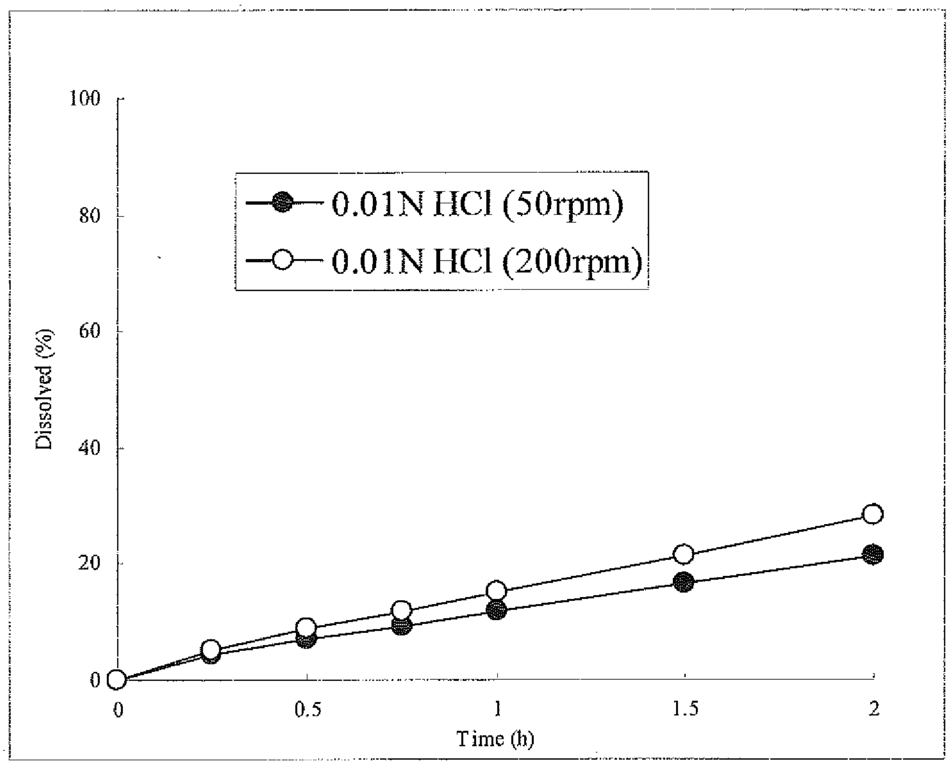


[Figure 8]

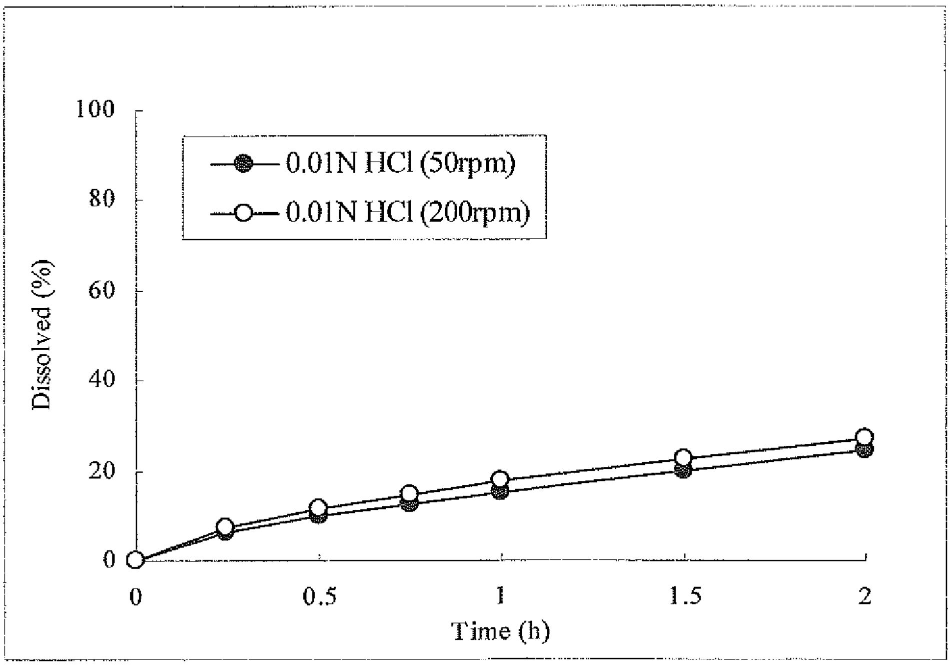

[Figure 9]

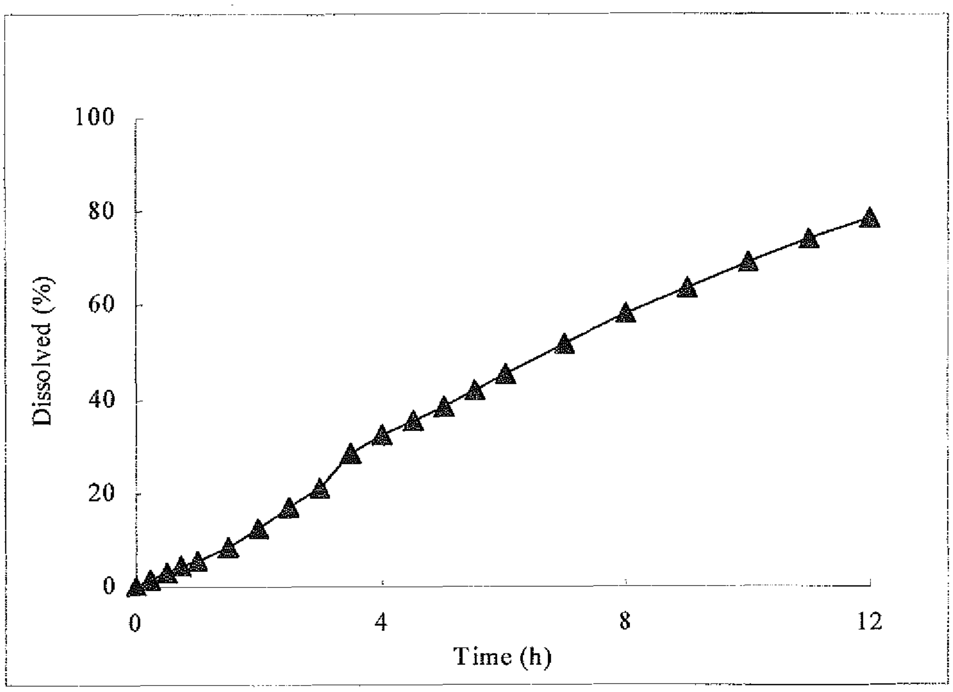


[Figure 10]

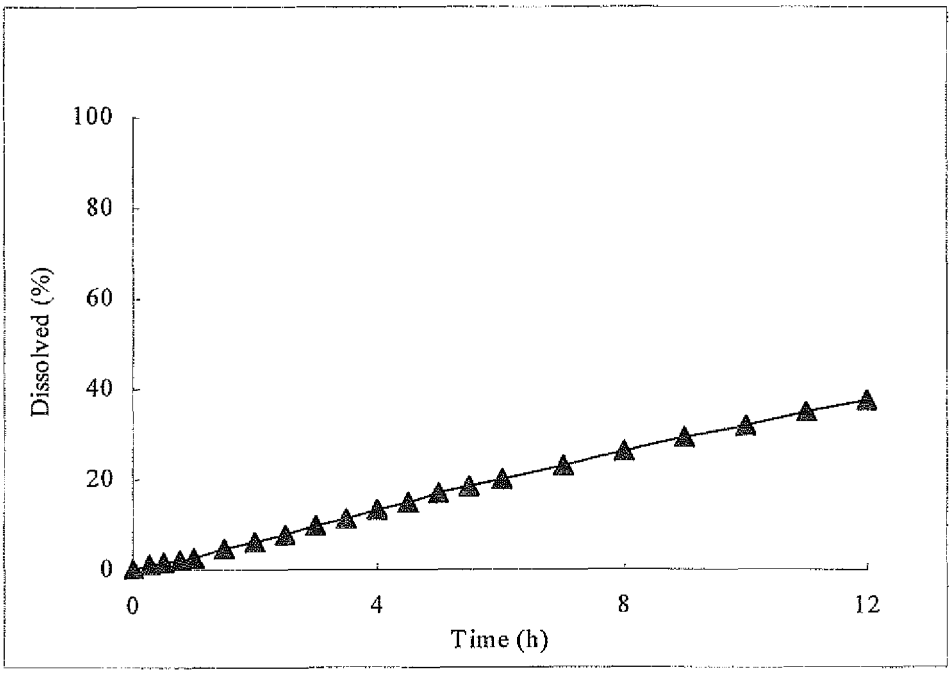

[Figure 11]

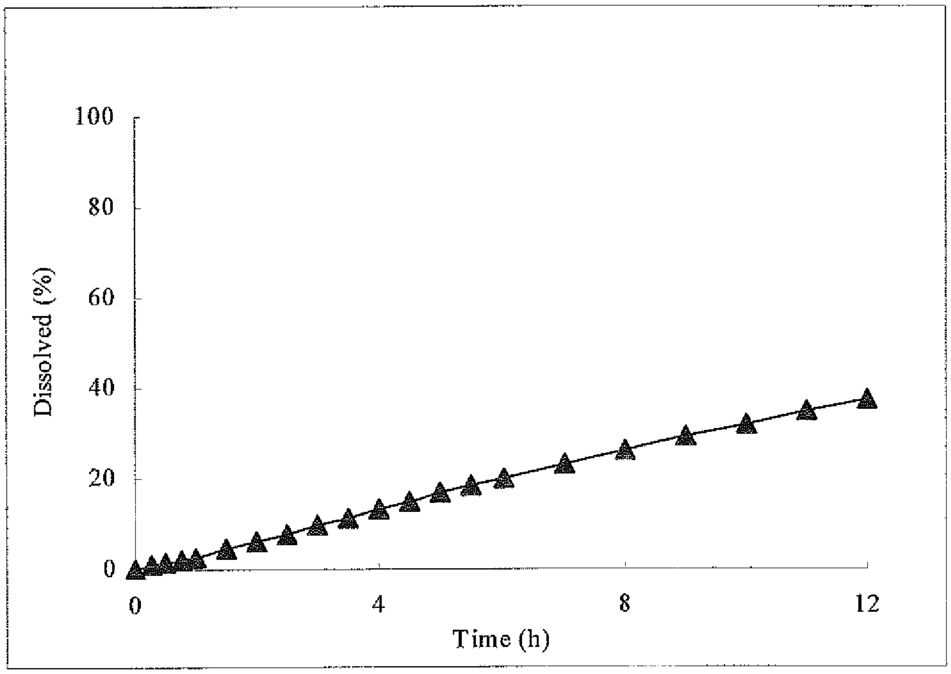


[Figure 12]

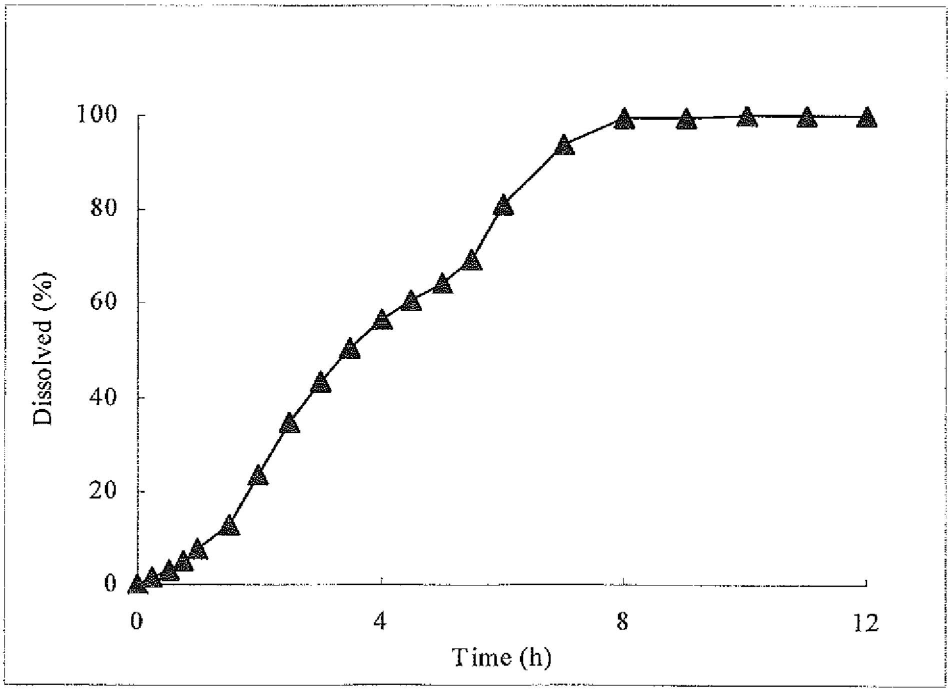

[Figure 13]

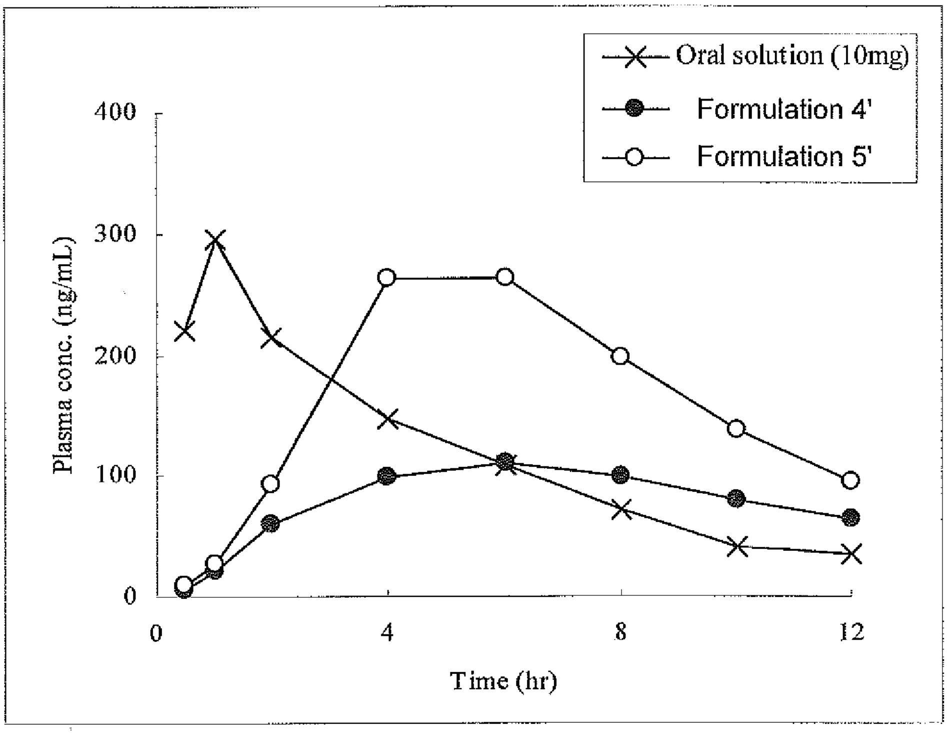
Oral solution (10mg)
Formulation 4'
Formulation 5'
400
300
200
100
0
Plasma conc. (ng/mL)
0
4
8
12
Time (hr)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004518676 A
- WO 2007031887 A2
- WO 2004058715 A
- WO 03000657 A
- WO 03000680 A
- WO 03016302 A
- WO 2003000657 A
- WO 2003000680 A
- WO 2003016302 A


### Non-patent literature cited in the description


- *WFO Drug Information,* 2004, vol. 18 (3), 260
- **SUSANNE R et al.** *J. Med. Chem.,* 2005, vol. 48, 5900-5908
- **D. KUBITZA et al.** Multiple dose escalation study investigating the pharmacodynamics, safety, and pharmacokinetic of Bay59-7939, an oral, direct Factor Xa inhibitor, in healthy male subjects. *Blood,* 2003, 102
- *WFO Drug Information,* 2006, vol. 20 (1), 38
- **ORWAT MJ ; LAM PYS et al.** Discovery of 1-(4-Methoxyphenyl)-7-oxo-6-(4-(2-oxopiperidin-1-yl)phenyl)-4,5,6,7-tetrahydro-1H-pyrazolo[3,4-c]pyridine-3-carboxamide (Apixaban, BMS-562247), a highly potent, selective, efficacious, and orally bioavailable inhibitor of blood coagulation factor Xa. *J. Med. Chem.,* 2007, vol. 50 (22), 5339-56
- *WFO Drug Information,* 2008, vol. 22 (3), 226-227
- **ZHANG P ; HUANG W ; ZHU BY et al.** Discovery of betrixaban (PRT054021), N-(5-chloropyridin-2-yl)-2-(4-(N,N-dimethylcarbamimidoyl)benzamido)-5-methoxybenzamide, a highly potent, selective, and orally efficacious factor Xa inhibitor. *Bioorg Med. Chem. Lett.,* 2009, vol. 19 (8), 2179-85
- **S. TAKEHANA et al.** *Japanese Journal of Pharmacology,* 2000, vol. 82 (1), 213
- **T. KAYAHARA et al.** *Japanese Journal of Pharmacology,* 2000, vol. 82 (1), 213
- *XVIIth Congress of the International Society for Thrombosis and Haemostasis,* 14 August 1999
- Generating greater value from our products and pipeline. *Aventis SA Company Presentation,* 05 February 2004
- *WFO Drug Information,* 2002, vol. 16 (3), 257
- **J. R. PRUITT et al.** *J. Med. Chem.,* 2003, vol. 46, 5298-5313
- **S. YOUNG.** *Medicinal Chemistry-12th RSC-SCI Symposium,* 07 September 2003
- **M. WILEY et al.** *ACS National Meeting,* 20040822